# EUROPEAN PATENT SPECIFICATION

(11) **EP 1 561 437 B1**
(45) Date of publication and mention of the grant of the patent: **28.10.2009**
(21) Application number: 04425074.4
(22) Date of filing: 06.02.2004
(51) Int. Cl.: A61F 2/24

(54) **External support for restoring competence to venous valves by traction of their intercommissural walls**
Externes Abstützelement zur Wiederherstellung der Funktionsfähigkeit von Venenklappen durch Zug an ihren Kommissurenwänden
Support extérieur pour rétablir la capacité des valvules du sinus veineux par traction de leur paroies intercommissurales

(43) Date of publication of application: 10.08.2005
(73) Proprietor: SANGOMED S.R.L., 00139 Rome (IT)
(72) Inventor: Camilli, Sante, 00139 Roma (IT)

(56) References cited:
- WO-A-02/26168
- WO-A-97/40755

## Description

This invention relates to a device for restoring competence to venous valves.

A device for restoring competence to venous valves is known from International Application No. WO 97/40755 (inventor ZUKOWSKI), published on 6th November, 1997, having the title *Device for Restoring Competence to Venous Valves,* which is the closest prior art.

ZUKOWSKI's device is based on the discovery that an external force applied onto a vein, at the level of the coapting surfaces of an incompetent, slackened valve of such vein, flattens the vein with the aim to extend the cusps of the valve laterally, taking up their slackening and bringing them into apposition, restoring the competence of the vein.

So ZUKOWSKI's device features a support for applying a compressive, corrective force to an incompetent vein.

ZUKOWSKI discloses a support having a pair of opposite compression membranes which are identical, and generally rectangular. The membranes may have an elliptic or arcuate configuration, or may be flat with curved ends; they are joined together by an integrally formed, intermediate hinge portion which, when the support has been positioned about a valve, generally extends axially with respect to the vein and is placed to be adjacent to one of the commissures where the cusps of the valve attached to opposite walls of the vein meet; the width of the hinge determines the compressive force applied by the support to the valve. The device is implanted by suturing the free edges of the two rectangular members. The sutures perform the same function as the hinge, and they can regulate the compressive force of the device on the venous walls by their degree of tightening.

Problems are associated with ZUKOWSKI's device.

ZUKOWSKI's device has two compression membranes which are continuous surfaces. Owing to such continuity, to implant it, it is necessary to completely clear the posterior wall of the vein from surrounding tissues, and from all eventual collateral veins. Moreover, for the same reason it is not suitable for curing an incompetent valve located at the confluence of another vein owing to the asymmetry and anatomical variability of the confluence itself.

Moreover, the compression from outside envisaged with ZUKOWSKI's device, actually does not ensure a contemporaneous increase of the intercommissural diameter *per se.* It is a disadvantage of ZUKOWSKI's device that its compressive action actually is not reliable as regards its corrective action.

In fact, it is not sure that applying antero-posterior compressive forces though reducing the antero-posterior diameter of the vein, forcedly determines an enlargement of the latero-lateral intercommissural diameter of the vein valve, because, really, *in vivo*: depending on the normally arising contraction of the muscular component of the venous wall during the surgical dissection of the vein from the surrounding tissues; depending on blood pressure inside the vein, and, depending on hormone-, or drug- induced contraction of the muscular component of the venous wall, the vein is not in its final condition, as really turns out to be hypothesised in the spirit of ZUKOWSKI's device, so that a calibration thereof is difficult and probably incorrect.

Moreover, the venous spasm directly consequent to surgical manipulation reduces the diameter of the blood-vessel circumferentially, so that one does not have the best apposition of the two valve cusps and therefore it is not possible to check the valve competence intraoperatively, i.e. during an operation.

It is the object of this invention to provide a device for restoring competence to venous valves that solves such problems.

Such an object is reached by an external support according to Claim 1.

The critical differences between the support of this invention and ZUKOWSKI's device are the following ones: (a) the inventive support acts by traction, as opposed to compression; (b) it is a device made as a frame shell, as opposed to continuous membranes; (c) the true working parts of it, in its traction work, are the sutures made by the surgeon, as opposed to ZUKOWSKI's device which only requires sutures to be closed.

Preferred embodiments are set forth in the subclaims.

It is also the subject matter of this invention a method for producing the external support taught by it, by an integrated fabrication according to Claim 9 or 10.

It is an advantage of the external support of this invention that it can be inserted after an extremely simple dissection of a posterior or deep passage tunnel. The surgeon only has to prepare two paths, without having to dissect the vein all over the length thereof. It can be inserted by easy-insertion branches of elongated material thereof through such passages. As the dissection is lesser than in ZUKOWSKI's device, the related venous spasm is lesser too. This facilitates the significance of the known 'milking manoeuvre', which is less dependable, or not feasible at all, under spasm.

This invention will be best understood based on the following detailed disclosure of non-limiting preferred embodiments thereof, given in reference to the enclosed drawings, wherein:
Figure 1 and Figure 1A respectively show a posterior and an anterior frame shell of the inventive external support, in a preferred embodiment;
Figure 2 shows the two frame shells of Figures 1 and 1A juxtaposed to each other in an assembled condition, defining an ellipse-like tunnel interior to receive a vein therein, and
Figure 3 schematically shows a vein restored to its competent condition by traction of its valve intercommissural walls by means of the inventive external support, once engaged by the sutures by the same intercommissural walls.

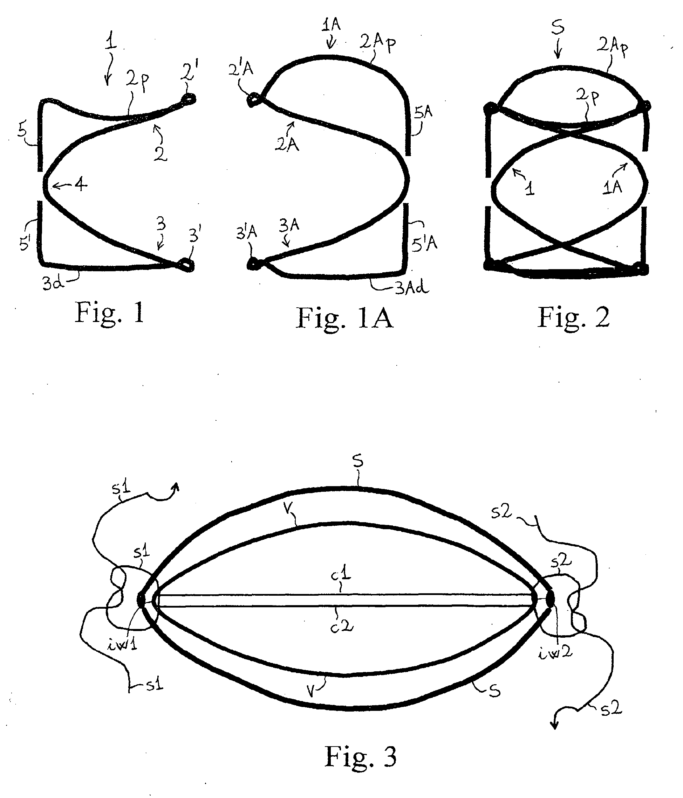

The external support includes two arcuate frame shells in an elongated material, respectively a posterior or deep frame shell 1 (see Figure 1) and an anterior or superficial frame shell 1A (see Figure 2). The external support is intended to be implanted about a vein longitudinally, i.e. along the length of the vein.

The two frame shells are to perform different functions.

Posterior frame shell 1 includes proximal and distal penetratingly shaped branches of the elongated material, for an easy insertion of the posterior frame shell behind the posterior wall of a vein through two paths prepared by the surgeon, without the need for the ligation of eventual collateral veins. The easy insertion branches according to the preferred embodiment depicted in the Figures are proximal and distal narrow bends 2, 3 of the elongated material, having apices shaped as eyelets 2', 3'. Narrow bends 2, 3 are centrally in continuity with each other by a large, central bend 4.

Anterior frame shell 1A has the same structure as posterior frame shell 1, with a proximal bend 2A with an eyelet 2'A; a distal bend 3A with an eyelet 3'A and a central bend 4A, but proximal and distal bends do not have to be shaped penetratingly.

The two frame shells have a respective longitudinal side of their arc-like shape intended to constitute a traction side. Such traction sides include respective longitudinal rectilinear side traction branches of the elongated material. According to the preferred embodiment, as depicted in Figures 1 and 1A, the traction branches are free-end branches 5, 5' for posterior frame shell 1, and 5A, 5'A for anterior frame shell 1A. The free-end traction branches 5; 5A; 5', 5'A respectively stem as continuations of proximal branches 2p; 2Ap, and of distal branches 3d; 3Ad of proximal 2, 2A and of distal 3, 3A bends of anterior 1 and posterior 1A frame shells, respectively. Central bends 4, 4A arrive onto the level of the longitudinal traction side of the arc-like shape of the frame shells. The free-end configuration for the traction branches serves to give a degree of elasticity for a better longitudinal flexibility thereof.

The two frame shells are to be juxtaposed to each other about a vein to be cured by the sides of their arcuate shapes - turning their concavities to each other - to assemble the support as depicted in Figure 3, defining an ellipse-like tunnel to receive vein V thereinto. The assembled external support S is shown in Figure 2. In this preferred embodiment, the frame shells are assembled by making free-end traction branches 5, 5' of posterior frame shell 1 to pass into eyelets 2'A, 3'A of anterior frame shell 1A, and making free-end traction branches 5A, 5'A of anterior frame shell 1A through eyelets 2', 3' of posterior frame shell 1. So the eyelets form means for reciprocal reversible mechanical connection of the frame shells.

The external support has a proximal section formed by proximal branches 2p, 2Ap, and a distal section formed by distal branches 3d, 3Ad, and a central section midway therebetween. The front-rear diameter of the support can decrease from the central section to the proximal and the distal section, to emulate the normal anatomic shape of natural valves, which favours the haemodynamic closing of the valves.

Proximal branch 2p of posterior frame shell 1 can be arcuate with its concavity outwards, as shown in Figure 1, whilst proximal branch 2Ap of anterior frame shell 1A can be arcuate with its convexity outwards, as shown in Figure 1A. In this way the external support, once assembled from frame shells 1, 1A as shown in Figure 2, has an inclined mouth 2p, 2Ap, which can conform itself to the confluence of two veins, at a proximal section thereof.

Referring to Figure 3, the inventive external support is mounted about a vein V with the longitudinal traction sides of the two frame shells respectively by opposite valve intercommissural walls iw1, iw2. It operates the restoring of the right apposition of the incompetent valve cusps by absorbing their slackening by traction. The traction force is applied onto the intercommissural walls iw1, iw2 of the venous valvular bulb by means of well known surgical sutures s1, s2 and is exerted along the intercommissural diameter - so along valve cusps c1, c2 - to dilate it, extending the cusps and absorbing their slackening. Surgical sutures s1, s2 respectively engage traction branches 5, 5'; 5A, 5'A to opposite intercommissural walls iw1, iw2. The surgical sutures can also engage central bends 4, 4A of the frame shells protruding onto the longitudinal traction sides of the frame shells. In Figure 3 the eccentric ellipse-like shape of vein V, which normally has a circular cross-section, under the action of the inventive support represents the effect of the traction of the latter once engaged by the sutures by the same intercommissural walls.

One frame shell or both frame shells can be laterally provided with hooks for engaging a venous wall by opposite valve intercommissural walls, to dilate the intercommissural diameter by traction. The hooks perform the same function as the sutures, i.e. they act the traction.

As an alternative embodiment, the frame shells can be integral to each other, being connected to each other by a hinge by a longitudinal side thereof.

The elongated material can be metal wire having good flexibility and elasticity, e.g. Nitinol®, or medical grade steel such as AISI 316.

However, the elongated material may also be e.g. a biocompatible plastic material, e.g. tetrafluoroethylene such as Teflon®, polypropylene, polyethylene.

According to this invention a method is envisaged for fabricating the external support taught by it in its integrated form, including an integrated fabrication step, such as cutting a foil or tube material, e.g. in a plastic material or in a metal, such as laser-cutting a metal foil; or such as moulding a plastic material.

In such a method a stage can be envisaged of fabricating the traction-acting hooks integratedly to the frame shells.

In such a method when starting from a foil material a step is envisaged of folding the foil material to create a longitudinal hinge.

When starting from a tube material, the tube is suitably shaped conformingly to the final arcuate, ellipse-like shape of the final support.

This invention has been disclosed referring to specific embodiments thereof, but it is to be understood that variations can be made thereto, without so departing from the scope of the appended claims.

## Claims

1. A traction-acting external support for restoring valvular competence to veins for implantation about a vein (V) longitudinally, i.e. along the length of the vein; including two arcuate frame shells, having longitudinal sides of an elongated material; said frame shells respectively being a posterior or deep frame shell (1) and an anterior or superficial frame shell (2); said posterior frame shell (1) including two branches (2, 3) of said elongated material penetratingly shaped for insertion of the posterior frame shell behind the posterior wall of the vein (V) through two paths; each frame shell (1, 2) including a respective traction side including longitudinal rectilinear side traction branches (5, 5'; 5A, 5'A); said two frame shells (1, 2) being conformed to be juxtaposed to each other about the vein (V) by their longitudinal sides; said traction sides being capable of being engaged to the vein (V) by opposite valve intercommissural walls (iw1, iw2) by means of surgical sutures (s1, s2) for dilating the intercommissural diameter of the vein by traction, so as to absorb the slackening of an incompetent venous valve by extending its cusps (c1, c2).

2. An external support according to Claim 1, wherein one or both said frame shells comprise hooks for engaging a vein by opposite valve intercommissural walls thereof, to dilate the intercommissural diameter thereof by traction.

3. An external support according to Claim 1 or 2, wherein said frame shells (1, 1A) are separate from each other, being able to be assembled to each other about a vein (V) by surgical sutures (s1, s2) .

4. An external support according to Claim 3, wherein said two frame shells are endowed with reciprocal reversible mechanical connection means, such as eyelets (2', 3'; 2'A, 3'A).

5. An external support according to Claim 1 or 2, wherein said two frame shells are integral to each other, being connected to each other by a hinge by a longitudinal side thereof.

6. An external support according to anyone of the preceding claims, wherein the front-rear diameter thereof longitudinally decreases from a central section thereof to a proximal section (2p; 2Ap) and to a distal section (3d, 3Ad) thereof.

7. An external support according to anyone of the preceding claims, wherein a proximal extremity section (2p) of said posterior frame shell (1) is arcuate with the concavity turned outwards, whilst a corresponding proximal extremity section (2Ap) of said anterior frame shell (1A) is arcuate with the convexity outwards.

8. An external support according to anyone of the preceding Claims, wherein said elongated material is a biocompatible material having good flexibility and elasticity, selectable among metals, e.g. Nitinol®, or medical grade steel, such as AISI 316, and plastics, e.g. tetrafluoroethylene such as Teflonc®, polyester such as Dacron®, polypropylene, polyethylene.

9. A method for fabricating the external support of Claim 5, including cutting a foil or tube-shell material, e.g. in a plastic material or in a metal, such as laser-cutting a metal foil.

10. A method for fabricating the external support of Claim 5 including moulding a plastic material.

## Patentansprüche

1. Äusserer Zugträger für der Länge nach einer Ader (V) Implantierung um der Ader die Klappefunktion zu wiederherstellen, der zwei bogenförmigen Längsseite eines ausgestreckten Material aufweisende Schalegerüste einschliesst; wobei die Schalegerüste ein Hinter- oder Tiefschalegerüst (1) beziehungsweise ein Vorderschalegerüst oder ein oberes Schalegerüst (2) sind und das Hinter- oder Tiefschalegerüst (1) ein zwei Zweige (2, 3) eines für Eindringen geformtes ausgestrecktes Material um das Hinter- oder Tieischalegerüst hinter der Hinterwand der Ader(V) mittels zwei Wege zu einstecken schliessen; jedes Schalegerüst (1, 2) eine dazugehörige Zugseite die die längsgeradlinigen Zugseitezweige (5, 5', 5A, 5'A) einschliesst umfasst; die zwei oben genannten Schalegerüste (1, 2) so geformt sind dass sie um die Ader (V) herum mit ihren Längseiten nebeneinander gestellt sind; wobei können die Zugseite mittels gegenüberliegender intercommissuraler Klappewände (iw1, iw2) mit chirurgischen Suturen (s1, s2) um mittels Zugaktions den intereommissuralen Aderdurchmesser zu erweitern mit der Ader (V) gebunden werden, so dass die Lockerung der venösen unfähigen Klappe mittels der Erweiterung der ihreren Segeln (c1, c2) aufgenommen wird.

2. Äusserer Zugträger nach dem Anspruch 1, wobei einschliesst/einschliessen ein der Schalegerüste oder beide Schalegerüste Haken für die Bindung der Ader mittels gegenüberliegenden interkommissuralen Klappewände um den intercommissuralen Durchmesser der Wände zu erweitern.

3. Äusserer Zugträger nach dem Ansprüche 1 und 2, wobei die oben genannte Schalegerüste (1, 1A) auseinander getrennt sind und die können um die Ader (V) herum mittels chirurgischen Suturen (s1, s2) zusammengebaut werden.

4. Äusserer Zugträger nach dem Anspruch 3, wobei die oben genannte Schalegerüste mit wechselseitigen reversibeln mechanischen Verknüpfungsmitteln als Knopflöcher (2', 3'; 2'A, 3'A) ausgestatten sind.

5. Äusserer Zugträger nach dem Ansprüche 1 und 2, wobei die oben genannte Schalegerüste miteinander einteilige und mit einem Scharnier entlang ihres Längsseite zusammengefügen sind.

6. Äusserer Zugträger nach jedem von der vorhergehenden Ansprüche, wobei der Vorderseite - Hinterseite Durchmesser entlang ihrer Längsseite von einem ihrem mittleren Abschnitt zu einem proximalen Abschnitt (2p; 2Ap) und zu einem distalen Abschnitt (3d, 3Ad) sich verringert.

7. Äusserer Zugtreger nach jedem von der vorhergehenden Ansprüche, wobei ein proximaler Endeabschnitt (2p) des obengenannten hinteren Schalegerüstes (1) mit der Konkavität nach Oben hin gelegen geschwungen ist, und dabei ein entsprechender proximaler Endeabschnitt (2Ap) des obegenannten Vorderschalegexüstes (1A) mit ihrem Konvexität nach aussem hin gelegen geschwungen ist.

8. Äusserer Zugträger nach jedem von der vorhergehenden Ansprüche, wobei das obengenannte Material ein biologisch verträgliches, gut biegsames und elastisches Material, das kann aus Metallen, nämlich Nitinol®, oder ein Stahl von medizinischem qualität wie AISI 316, und ein Kunststoff wie Tetrafluoroethylen, beispielweise Teflon®, ein polyester wie Dacron, Polypropylen, Polyethylen ausgewählt werden ist.

9. Verfahren um den äusseren Zugträger des Anspruch 5 zu herstellen, wobei einschliesst das genannte Verfahren den Schnitt des Blattmaterial oder einer röhrenförmigen Schale wie beispielweise in einem plastischen Material oder in einem Metall, wie das Verfahren wobei ein Metallblatt mittels eines Laser geschnitten ist.

10. Verfahren um den äusseren Zugträger des Anspruch 5 zu herstellen, wobei einschliesst das genannte Verfahren das Pressen eines plastisches Materials.

## Revendications

1. Support extérieur fonctionnant par traction pour rétablir la compétence valvulaire des veines pour l'implantation autour d'une veine (V) longitudinalement, c'est-à-dire le long de la longueur de la veine; comprenant deux coques à châssis arquées, ayant leurs côtés longitudinaux d'un matériel allongé; les dites coques à châssis en étant respectivement une coque à châssis postérieure ou profonde (1) et une coque à châssis antérieure ou superficielle (2); la dite coque à châssis postérieure (1) en comprenant deux branches (2, 3) du dit matériel allongé façonnées de forme pénétrante pour l'insertion de la coque à châssis postérieure derrière la paroi postérieure de la veine (V) à travers deux parcours; chaque coque à châssis (1, 2) en comprenant un respectif côté de traction comprenant des branches de traction latérales rectilignes longitudinales (5, 5' ; 5A, 5'A); les dites deux coques à châssis (1, 2) en étant conformées pour être juxtaposées l'une à l'autre autour de la veine (V) par leurs côtés longitudinaux; les dits côtés de traction en étant capable d'être engagés à la veine (V) par des parois intercommisurales valvulaires opposées (iw1, iw2) au moyen de sutures chirurgicales (s1, s2) pour dilater le diamètre intercommissural de la veine par traction, de sorte à absorber le relâchement d'une valvule veineuse incompétente en étendant les cuspides (c1, c2) de celle-ci.

2. Support extérieur selon la Revendication 1, dans lequel l'une des ou les deux dites coques à châssis comprennent des crochets pour engager une veine par des parois intercommissurales valvulaires opposées de celle-ci, pour dilater le diamètre intercommissural de celle-ci par traction.

3. Support extérieur selon la Revendication 1 ou 2, dans lequel les dites coques à châssis (1, 1A) sont séparées l'une de l'autre, en étant capable d'être montées l'une à l'autre autour d'une veine (V) par des sutures chirurgicales (s1, s2).

4. Support extérieur selon la Revendication 3, dans lequel les dites coques à châssis sont douées de moyens de connexion mécanique réversible réciproque, tels que des oeillets (2', 3' ; 2'A, 3'A).

5. Support extérieur selon la Revendication 1 ou 2, dans lequel les dites deux coques à châssis sont solidaires l'une de l'autre, en étant connectées l'une à l'autre par une charnière par un côté longitudinal d'elles-mêmes.

6. Support extérieur selon l'une quelconque des revendications précédentes, dans lequel le diamètre antéro-postérieur de celui-ci décroît longitudinalement d'une section centrale de celui-ci à une section proximale (2p ; 2Ap) et à une section distale (3d, 3Ad) de celui-ci.

7. Support extérieur selon l'une quelconque des revendications précédentes, dans lequel une section d'extrémité proximale (2p) de la dite coque à châssis postérieure (1) est arquée avec sa concavité tournée à l'extérieur, tandis qu'une section d'extrémité proximale correspondante (2Ap) de la dite coque à châssis antérieure (1A) est arquée avec sa concavité à l'extérieur.

8. Support extérieur selon l'une quelconque des revendications précédentes, dans lequel le dit matériel allongé est un matériel biocompatible ayant une bonne flexibilité et élasticité, sélectionnable parmi les métaux, par exemple Nitinol®, ou un acier de qualité médicale, tel que AISI 316, et les matières plastiques, par exemple tétrafluoroéthylène tel que Teflon®, un polyester tel que Dacron®, polypropylène, polyéthylène.

9. Méthode pour fabriquer le support extérieur de la Revendication 5, comprenant le couper un matériel en feuille ou en coque de tube, par exemple en une matière plastique ou en un métal, tel que le couper à laser une feuille métallique.

10. Méthode pour fabriquer le support extérieur de la Revendication 5, comprenant le mouler une matière plastique.
